(19) Europäisches Patentamt
European Patent Office
Office européen des brevets

(11) **EP 4 537 778 A1**

(12) **EUROPEAN PATENT APPLICATION**

(43) Date of publication:
**16.04.2025 Bulletin 2025/16**

(21) Application number: **23203485.0**

(22) Date of filing: **13.10.2023**

(51) International Patent Classification (IPC):
***A61B 18/18*** (2006.01)

(52) Cooperative Patent Classification (CPC):
**A61B 18/18;** A61B 2018/00476; A61B 2018/0066;
A61B 2018/1807

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
- **VAN ZUTPHEN, Martijn
  5656 AG Eindhoven (NL)**
- **KINGMA, Harmen Andries
  5656 AG Eindhoven (NL)**

- **FLINSENBERG, Ingrid Christina Maria
  5656 AG Eindhoven (NL)**
- **JANSEN, Marjolein Yvonne
  5656 AG Eindhoven (NL)**
- **BONG, Leonita
  5656 AG Eindhoven (NL)**
- **NETO, Sofia Inês
  5656 AG Eindhoven (NL)**
- **NENGERMAN, Joost Willem Frederik
  5656 AG Eindhoven (NL)**

(74) Representative: **Philips Intellectual Property &
Standards
High Tech Campus 52
5656 AG Eindhoven (NL)**

(54) **LIGHT-BASED HAIR OR SKIN TREATMENT**

(57) An intense pulse light, IPL, system is for delivering IPL light to a user's skin, and comprises a hand-held IPL device with a motion sensor for sensing motion of the IPL device, and a processor (which may be part of the hand-held device or remote or partly remote and partly in the hand-held device) for processing the motion sensor output and timing of delivery of light flashes. The processor determines a step size of movement of the IPL device and generates feedback information to the user relating to the step size. This information is used for coaching the user in the most effective use of the IPL device.

FIG. 2

EP 4 537 778 A1

**Description**

FIELD OF THE INVENTION

This invention relates to hair or skin treatment devices, in particular intense pulsed light, IPL, devices.

BACKGROUND OF THE INVENTION

For hair removal, light-based epilators are well-known. People use hair epilators or depilators to remove unwanted hair. Typical target areas for women are the face, armpit, arm, leg, bikini line, and body. Men use light-based epilators also on the chest and back.

Light-based hair removal devices are based on the filtered output of a flashlamp, known as Intense Pulsed Light or IPL. IPL devices also have the advantage that they enable various skin treatments to be combined with a hair removal mode.

It is known to provide an IPL device with an associated app, which is used to provide coaching features. During first-time use of the IPL device, it asks the user to measure the length of their body part in question (e.g., the leg) and then calculates the required number of flashes (based on the treatment window width). After this, the app asks the user to count the actual number of flashes for one stroke, and then reports a coverage percentage.

This coaching approach is not considered very "smart" since it involves user input to calculate coverage. It requires a certain effort by the user (like getting a measuring tape) which will discourage many users.

Smarter executions of the length-measuring and flash counting approach have been proposed. For example, it is possible to measure the length of the leg using a photo, and to automatically count flashes using analysis of the sound that the device makes. This is all possible through the application of machine learning technologies. However, this still requires the user to change their normal routine significantly and thus this still requires more user interaction than is probably acceptable. Moreover, these approaches only give an overall (average) coverage per treatment stroke and cannot detect where on the body part the coverage was good or bad.

It would be of interest to users to enable an automatic step size measurement to be implemented using sensors. Measuring the actual step size between each flash (or averaged over a few flashes) using sensors would allow coaching concepts which require less user interaction and would enable more granular feedback regarding the performance over the length of the treatment strokes.

SUMMARY OF THE INVENTION

The invention is defined by the claims.

According to examples in accordance with an aspect of the invention, there is provided an IPL system for delivering IPL light to a user's skin during IPL treatment, comprising:

>   an IPL device comprising:

>>      a light source;
>>      a light delivery window;
>>      a controller for controlling the light source to deliver light flashes to the light delivery window and for reporting a timing of the light flashes as light flash timing; and
>>      a motion sensor for sensing motion of the IPL device;

>   a processor for processing the motion sensor output and the light flash timing; and
>   an output interface,
>   wherein the processor is configured to:

>>      derive a step size of movement of the device between light flashes from the motion sensor output and the light flash timing; and
>>      control the output interface to provide feedback information to the user relating to the step size.

This IPL device provides information to the user about the step size they have applied between flashes of the light source. In particular, this information relates to how well the user has performed a treatment using the device. For example, the step size between flashes should correspond to the length of the light delivery window (in the direction of movement) so that there is continuous skin coverage with no gaps and no overlap. In this way, coaching can be provided to assist the user in achieving the most optimal coverage and also to improve handling of the device.

The IPL device for example has a trigger button, which the user actuates to generate a light flash. The user then moves the device to the next skin location to perform the next light flash. Thus, the reporting of the timing of the light flashes may then be derived from the trigger actuation and/or from the light source actuation signal.

The processor may be part of the IPL device, which is a hand-held device, or it may be remote from the hand-held device so that more processing power is available. Similarly, the output interface may be part of the hand-held device or it may be remote from the hand-held device (such as a mobile phone or tablet) so that a richer user interface experience may be obtained.

The processor is for example configured to assume a zero movement of the IPL device during the light flash. It is difficult to calculate the linear acceleration perfectly. This linear acceleration is the real acceleration after subtracting the (very dominant) gravity vector that is always present in 3-axis acceleration sensors. In practice, for low-cost motion sensors (e.g., IMU sensors) there is always a small remaining acceleration (offset) in the calculated linear acceleration, even at standstill. This small offset, after being integrated twice, causes a drift in the distance calculations, resulting in incorrect step size estimations. However, by assuming zero velocity during flashes, the offset can be calculated and subtracted from the data. For example, at every flash, an offset/drift estimation may be updated. This enables a significant reduction of step size estimation errors.

The processor is for example configured to control the output interface to provide user coaching in respect of the correct amount of movement between flashes to achieve skin coverage. It is noted that the correct amount of movement may depend on the orientation with which the IPL device is used, for example if it has a rectangular light delivery window.

The IPL device for example comprises a skin detection sensor, wherein the processor is further configured to detect when the device is placed on the skin. The skin detection is used to provide a safety function so that the light is only delivered when the device is against the skin. In addition, it can be used to enable additional information to be derived about the user's performance of the IPL treatment, and to detect when the user transitions from one line of treatment to a next line of treatment. In this way, movement between the lines of treatment (with the IPL device lifted off the skin) will not be counted as a step and hence will not distort the step size information.

The processor is for example configured to control the output interface to provide user coaching in respect of one or more of:

correct application of the light delivery window to the skin during movement between flashes (i.e., the window is held against the skin).

correct orientation of the light delivery window to the skin during movement between flashes (i.e., there is not excessive rotation);

sufficiently small movement of the user during the IPL treatment (i.e., there is not excessive movement of the body part to which the IPL treatment is applied).

It is preferred that the user maintains the IPL device against the skin during a line of treatment, and this helps the motion tracking to provide accurate step size information. Reasonably accurate results can however be obtained if the deviation from the skin is not too large (such as a mm range). Thus, use of skin detection also enables additional coaching to be provided to use the IPL device in the most effective way, and in a way which provides the most accurate feedback and most suitable coaching.

The processor is for example configured to:

derive a 3D linear acceleration by removing a gravity component from the motion sensor output;

integrate the 3D linear acceleration to derive a 3D velocity assuming a zero movement of the IPL device during the light flashes;

integrate the 3D velocity to derive a step size estimate between flashes.

This is a purely mathematical approach to obtaining the step size estimate, but using the assumption, as discussed above, of no movement during the flash.

The processor may be further configured to provide the step size estimates to a machine learning algorithm thereby to obtain a refined step size estimate. The machine learning algorithm is for example trained using ground truth data obtained by accurate location tracking, e.g. using camera-based motion tracking systems. The combination of mathematical processing followed by machine learning refinement enables a reduced size training dataset for the machine learning.

An improvement in the accuracy of the resulting step size estimate is obtained due to the fact that the machine learning model can be trained to detect patterns in (a sequence) of signal segments which are not clearly visible/obvious for a human. Also, such a model can apply (non-linear) techniques like scaling, adding offsets and limiting step size estimates based on the ground truth data with the aim of lowering the overall estimation error.

The motion sensor for example comprises a 6-axis inertial monitoring unit, IMU. The system may however further comprise a second motion sensor for detecting motion relative to the user's skin. This may for example comprise an optical tracking system. This additional motion sensing may then be used to improve the anchoring of the main motion sensor signals to movement of the IPL device relative to the skin.

The invention also provides a method of processing a motion sensing signal and a light flash timing signal obtained during treatment with the IPL device, the method comprising:

deriving a step size of movement of the device between light flashes from the motion sensor output and the light flash timing signal; and
controlling an output interface to provide feedback information to the user relating to the step size.

The information is for example in the form of coaching to assist the user in obtaining the most optimal technique when using the IPL device.

The method may comprise assuming a zero movement of the IPL device during the light flash.

The method may comprise controlling the output interface to provide user coaching in respect of the correct amount of movement between flashes to achieve skin coverage.

The method may comprise receiving a skin contact detection signal obtained during the IPL treatment and controlling the output interface to provide user coaching in respect correct application of the light delivery window to the skin during movement between flashes.

The method may comprise:

deriving a 3D linear acceleration by removing a gravity component from the motion sensor output;
integrating the 3D linear acceleration to derive a 3D velocity assuming a zero movement during the light flashes;
integrating the 3D velocity to derive a step size estimate between flashes.

In addition to this mathematical processing, the method may comprise providing the step size estimates and intermediate mathematical processing results to a machine learning algorithm thereby to obtain a refined step size estimate.

The invention also provides a computer program comprising computer program code which is adapted, when said program is run on a computer, to implement the method as defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an IPL device;
Fig. 2 shows an IPL system combining a hand-held device and remote device for remote processing and/or feedback;
Figs. 3A and 3B show the steps performed by the motion sensor processing algorithm;
Fig. 4 shows a device speed calculation principle;
Fig. 5 shows a 3D step size estimation principle; and
Fig. 6 shows the beneficial effect of averaging step sizes.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an intense pulse light, IPL, system for delivering IPL light to a user's skin, comprising a hand-held IPL device with a motion sensor for sensing motion of the IPL device, and a processor (which may be part of the hand-held device or remote or partly remote and partly in the hand-held device) for processing the motion sensor output and timing of delivery of light flashes. The processor determines a step size of movement of the IPL device and generates feedback information to the user relating to the step size. This information is used for coaching the user in the most effective use of the IPL device.

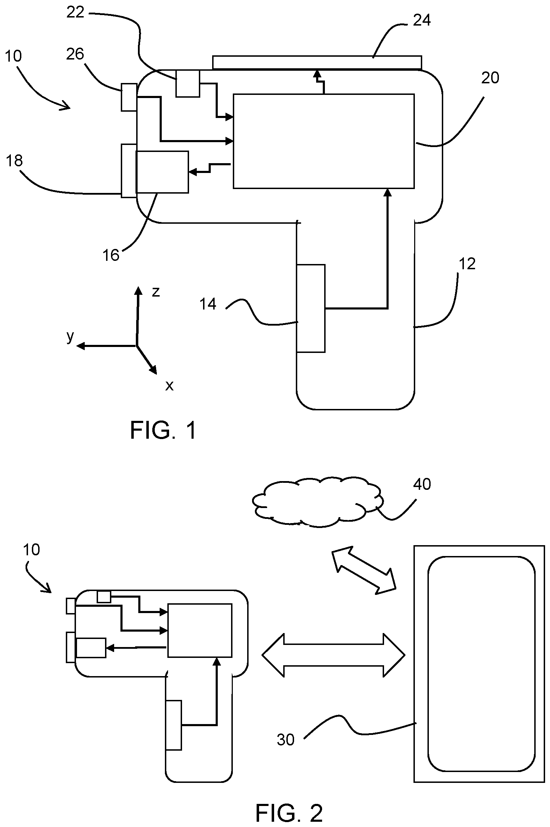

Fig. 1 shows an IPL device 10, in the form of a hand-held device having a handle 12 with a trigger 14, a light source 16 providing light through a light delivery window 18. A controller 20 controls the light source 16 to deliver light flashes to the light delivery window 18.

The controller also reports the timing of the light flashes (as "light flash timing"). This light flash timing may instead or additionally be derived from activation of the trigger 14. A motion sensor 22 senses the motion of the IPL device.

The motion sensor for example comprises a 6- axis Inertial Monitoring Unit, IMU. A 6-axis IMU measures 3-axis accelerations and 3-axis gyroscope signals. The IMU is a small surface mount device (SMD) component which can be soldered directly to the printed circuit board (PCB) of the device. The IMU should be as close as possible to the treatment location, i.e., the center of the light output (treatment) window. Movements measured at the location of the IMU can be translated to movements at the treatment location using a coordinate transformation, however small (orientation) errors will be translated to significant position errors if there is a large distance between the IMU and the treatment location.

Inertial Measurement Units (IMUs) are used in many situations where movement of devices must be detected/measured. For instance, a step counter in a smart watch, or pick-up detection in a smartphone. It is also generally known to use IMUs for providing guidance in hand-held personal care devices, such as detecting the type of motion of a shaver in use (EP3621775), and to generate heat maps of treatment parameters, such as a face map showing where on the face the user applied correct or incorrect shaving pressure (EP3938155). Algorithms based on IMU data are also known in the field of oral healthcare to provide tooth brushing feedback and guidance (WO2017/002004 and WO2016/174621)

The monitoring of movement between flashes using an IMU gives a low-cost implementation, since the IMU can be implemented in the handle of the device without needing to be in the head that makes contact with the skin. In this way, the same sensor may be used when different attachments are selected (an IPL device typically comes with several types for different body areas).

However, calculating device location based on 3D acceleration data from an IMU is not self-evident. There are several challenges to overcome:

(i) IMUs measure acceleration (in $m/s^2$) not position/displacement (in mm). It is possible to integrate acceleration twice to derive a distance travelled, but due to the noisy nature of (inexpensive) IMUs and the fact that they usually have an offset/bias, this leads to inaccurate distance estimations, especially when measured over longer time durations (where several seconds will already be considered a long duration).

(ii) The acceleration that an IMU measures (in 3 directions) will also contain the gravity vector, which is very dominant compared to the low "real" accelerations. The gravity vector can be removed using a low pass filter, but this introduces lag.

(iii) Even after overcoming the above limitations, it is only possible to measure device displacement, not the movement relative to the body. If, during the movement of the device over the body e.g., leg, the user also moves the leg itself, this leg movement cannot be measured. and the system will measure the sum of both movements. Without an IMU on the leg, it is (in principle) impossible to discriminate between these two movements.

(iv) Even if it is possible to measure relative displacement over the body part (e.g. leg), it is still not possible to know exactly where on the body part the device is positioned. At least the starting point of the movement must be known for this.

The preferred implementation of the invention described below, and in particular an algorithm which processes the IMU sensor data, addresses these issues. For this purpose, a processor is used to process the motion sensor output and the light flash timing. In the example of Fig. 1, the controller and processor are shown as a single entity 20 which performs all processing and control functions. An IPL device itself contains a microcontroller, which dependent on the algorithm used could already be powerful enough. Thus, in one implementation, a complete guidance algorithm may be hosted on the IPL device controller, when a sufficiently powerful controller is selected.

However, as will be clear from Fig. 2, the light source control and data processing functions may be split between two or more different entities. For example, the local processor may have insufficient processing power to incorporate a machine learning algorithm, so that more processing power is needed than an embedded microcontroller (e.g., ARM-based) can typically deliver.

The device of Fig. 1 also has an output interface 24, such as a screen and/or loudspeaker. The output interface to the user may instead or additionally by provided by a remote device with which the hand-held device communicates as also shown in Fig. 2. A skin sensor 26 is used to detect skin contact. It may comprise a single sensor, or two or more sensors to detect that the light output window is flat against the skin.

In use of the device, a user applies the light output window to the skin and presses the trigger 14 to deliver a pulse of light. The user then slides the device over the skin to the next treatment location, and applies the trigger to deliver the next flash of light. It is also possible in some devices to keep the trigger depressed during the line treatment. It will then automatically flash every time a capacitor in the device is charged enough, which is typically 1-2 seconds depending on the setting. The user may then just move the device to the next spot, wait for the next flash, and move on again A line of flashes is applied along the length of a body part, before a next line is started.

The use of such a device is not totally straightforward since the flashes of light should be applied in a continuous line (to avoid untreated gaps) and in non-overlapping manner (to avoid over exposure). Thus, it is useful to provide user coaching to assist in the correct use of the IPL device.

The processor 20 derives a step size of movement of the device between light flashes from the motion sensor output and the light flash timing. This step size information is then used to control the output interface 24 to provide feedback information to the user relating to the step size, in particular in the form of a report on the quality of the way the device is being used and/or coaching.

Fig. 2 shows an IPL system comprising an IPL device 10, again with a handle, trigger, light source, light delivery window and controller. The controller may perform some of the sensor data processing as well as controlling the light source, and some of the sensor data processing can be performed remotely, for example on an external device such as a smartphone 30 or in the cloud 40. Data can be sent to the external device via wireless communication, such as WiFi or Bluetooth. The smartphone can for example forward the data (perhaps after some light pre-processing) to a server that runs in the cloud. The results of the calculations are then sent back to the smartphone or the IPL device for displaying them to the user.

The output interface may also be provided at the external device as well as, or instead of, at the hand-held device. Thus, the output interface may comprise the screen (and microphone) of a mobile phone or tablet.

As explained below, the sensor data processing may comprise a combination of a mathematical algorithm and a machine learning algorithm, By way of example, the mathematical algorithm may be hosted locally in the hand-held device and the machine learning algorithm may be hosted remotely. However, any desired division of processing functions may be envisioned.

The processor has various inputs, in addition to the IMU motion sensing data, in particular the status of the skin detection sensor (e.g. skin detected yes/no or raw skin detection sensor data), the status of the trigger (on/off), the timing instants at which the lamp flashed and a timestamp of the IMU measurements.

The processing of the IMU data (and other received sensor data) involves use of an algorithm by the processor. The algorithm translates the raw IMU sensor data to a step size estimation (i.e., a distance moved between flashes of the device, and hence the distance along the skin that the device has been moved), detects when the user moves to a new stroke, and in a preferred implementation of the algorithm identifies detected movements which the algorithm cannot reliably generate an accurate step size or the user employs techniques that are not recommended for optimal results. These will be termed "outlier use case" movements. Thus, they are movements made with the device which cannot be tracked accurately, or the expected treatment result can be suboptimal. For example, if there is a long time between flashes, the step size estimation may be too inaccurate (due to the double integration explained below). A stamp-and-flash motion or horizontal/vertical rotations during a stroke will result in a suboptimal treatment.

As explained below, the algorithm may combine mathematical processing of the data and machine learning processing. For the machine learning aspect, the algorithm is trained using ground truth data from a separate, accurate measurement system such as an optical motion tracking system.

The purely mathematical aspect of the algorithm involves combining sensor data, performing coordinate transformations, integrating the (linear) acceleration, and performing other mathematical functions such as calibration to improve the result. However, the step size calculated using this approach will typically have errors because the system is not perfect. One cause is that the sensor exhibits noise and bias or offset. Another cause is that the user can move the body part together with the IPL hand-held device. This effectively results in no movement with respect to the body part, but will be registered as movement by the motion sensor.

By training a machine learning (ML) algorithm, such as a neural net, the accuracy of the result from pure mathematical processing can be significantly improved. The model is better able to separate the noise from the actual signal than traditional methods can achieve.

The algorithm produces results such as last step size, new stroke or outlier-use case detected. This needs to be transformed into something that is relevant and understandable to the end user. For instance, it could be translated in an average coverage percentage of the last few flashes, together with advice about any improvement that may be suggested. The feedback can be displayed in the smartphone app, but consumers might not like the idea of having to watch their phone screen all the time during the treatment. Therefore, the feedback could also be given via sound coming from the smartphone app (e.g., a computer-generated voice) or via a visual indicator on the IPL device itself (e.g. a light indicator or a small display). Thus, a local output interface 24 and a remote output interface (the smartphone) may both be used in combination.

Optimizing the mathematical function to detect step size and training a machine learning part of the algorithm both require ground truth data, i.e., the real distance that the device has travelled with respect to the body part (e.g. leg). For capturing such ground truth data, commercially available optical motion tracking systems can be used. These systems are for example well known for recording motion of actors for animation movies, or for performing motion analysis with athletes. This system for example uses reflective markers (balls) attached to the parts that are to be tracked. By attaching several of these markers to the IPL device and to the body part being treated, both the position and orientation of these objects can be tracked. Known systems can capture the location and orientation of objects with high accuracy and with high sample rates (e.g., 120 Hz), require only minimal modifications to the devices, and do not have a large impact on the routine that needs to be followed by users. Therefore, such systems are ideal for generating the ground truth for this application as part of the algorithm development and training. In developing the system, training data acquisition was performed using external respondents in a controlled environment.

In use of the device (after the development is complete) the system captures skin sensor data and IMU data and processes that to generate guidance and coaching data. The IPL device is used by moving the device along a line, called a stroke, and then moving to a new stroke. Typically, each stroke follows the same direction, e.g., the bottom of the leg towards the top of the leg. Movement between the strokes is not monitored, because the distance is too great for the new start position to be determined accurately.. Each stroke involves a sequence of flashes, with the movement of the device between two treatment flashes called a step. Each step contains a segment of:

> IMU sensor sample times
> IMU data
> skin contact sensor data (which may be one or more sensor signals)
> Lamp flash data
> Trigger button data

It is noted that only the lamp flash data is needed for the flash timing (the trigger button data is not needed). However, the trigger button signal is also beneficial. For example, the trigger may be used for skin contact searching and for zero velocity measurement. If the user presses the trigger button while not having skin contact (and therefore not resulting in a lamp flash) it can be assumed that the device is not moving. The trigger button can thus be used to give additional information about user behavior and device usage.

By partitioning the data into steps, an assumption is made about the sensor data, namely that the device is firmly on the skin and not moving when flashing. Therefore, the movement speed during a treatment flash is zero. This enables implementation of an integration and measurement error correction in the device speed measurement algorithm. Of course, this correction mechanism will only work when the device is indeed not moving during flashing. Based on current experience, this is true in the majority of cases.

The step data segments are input to the guidance data generation algorithm. Movement caused by moving the treated body part while the device is placed on the skin will cause displacement measurement errors which are very difficult, if not impossible, to compensate for. Therefore, the body part should be kept as still as possible during a treatment stroke. For similar reasons, the device should also not be lifted too far off the skin during a treatment stroke. It also means the reference of the device movement with respect to the skin is lost, in particular the body part may move relative to the hand-held device when it is lifted. The resulting step size estimate will be wrong in this case.

The system can provide real time guidance about the performance during a stroke and it can provide an analysis of a finished treatment. For the real time guidance, between strokes there is no guidance information other than that the previous stroke has ended.

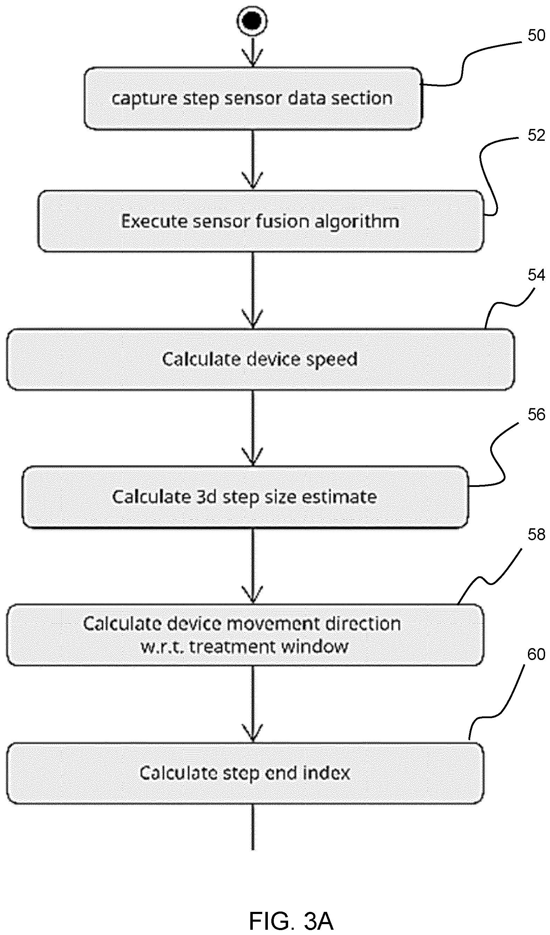

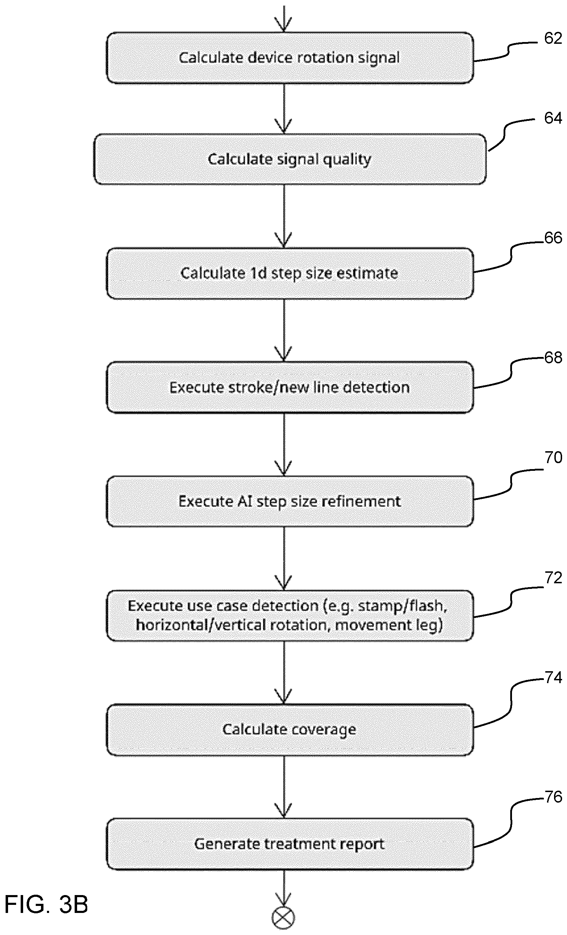

Figs. 3A and 3B show the steps performed by the algorithm. The steps in Fig. 3B follow from the steps in Fig. 3A.

In step 50 the sensor data (listed below) is captured for the current step. The skin sensor data is used to detect when the device is placed on the skin. As soon as skin is detected and the lamp flashes in response to trigger actuation, the capture of a step data section starts. The next lamp flash indicates the end of the current step data section. The following data is captured:

> Time data
> Accelerometer sensor data: An array of raw 3D acceleration sensor samples
> Gyroscope sensor data: An array of raw 3D gyroscope sensor samples
> Skin sensor data
> Lamp state data
> Trigger state data

This data is captured at a sample-rate for example of tens of Hz such as 50Hz, which is the IMU sample rate.

In step 52, a sensor fusion algorithm is executed. This involves calculating the 3D linear acceleration signal of the step segment. This is basically the acceleration signal minus the gravity component. By way of example, the Madgwick algorithm may be used (Sebastian Madgwick. An efficient orientation filter for inertial and inertial/magnetic sensor arrays.

April 30, 2010. http://www.x-io.co.uk/open-source-imu-and-ahrs-algorithms).

The linear acceleration signal is used as the input for the device speed estimation algorithm which is applied in step 54.

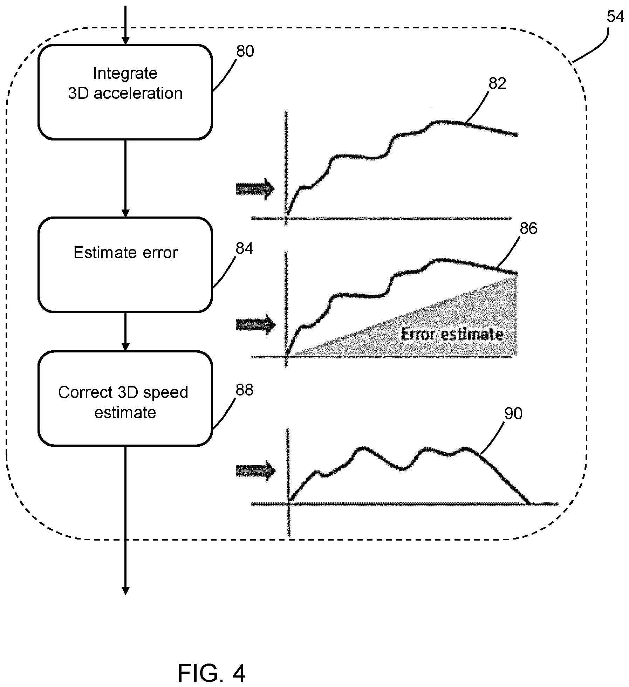

The device speed calculation principle is shown in Fig. 4. As is well known, speed can be calculated by integrating a linear acceleration signal, shown as step 80. In an ideal theoretical situation this would result in a perfect speed signal. However, a combination of IMU measurement errors in combination with integration errors will cause a large signal drift as shown by graph 82 showing an uncorrected speed signal which includes a drift, which increases over time. This drift needs to be filtered out as much as possible.

By assuming that the device speed is 0 during a treatment flash, a 3D speed estimate can be calculated. An error is estimated in step 84, and this error is shown in graph 86 as the shaded area. Removing this error component in step 88 provides a 3D speed estimate shown by plot 90.

An x/y/z integration step error estimate is calculated based on the x/y/z speed values of the last sample of the uncorrected speed signal (using the zero-speed-during-flash assumption). These x/y/z step error estimates are used to calculate and subtract the error signal from the speed signal.

Returning to Fig. 3A, a 3D step size estimate can then be obtained in step 56.

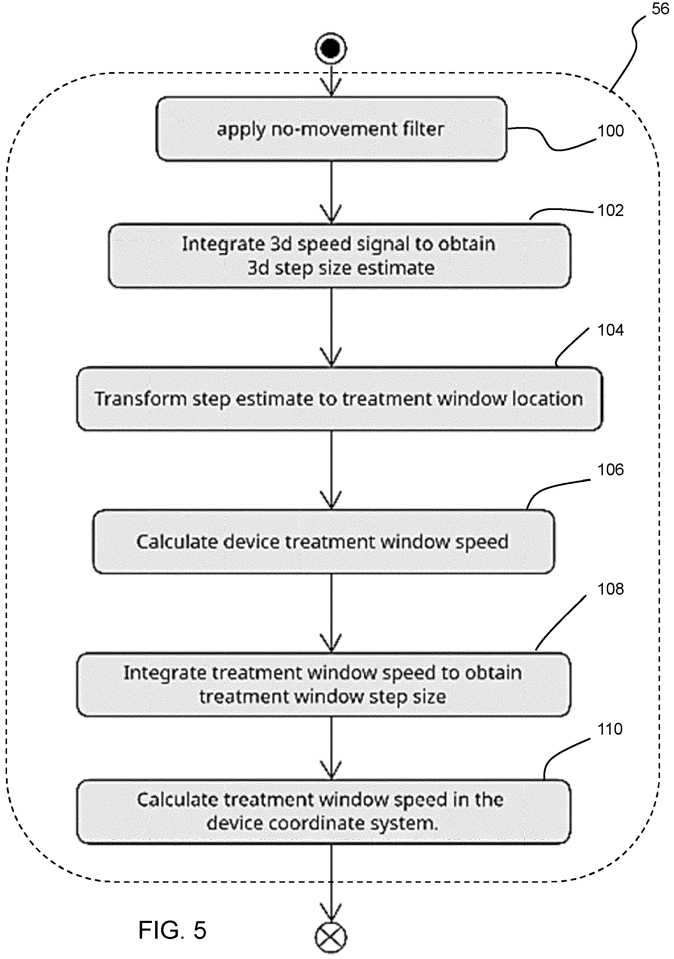

The 3D step size estimation is shown in Fig. 5.

The first step 100 is to apply a "no-movement" filter. By filtering out the parts of the signal which do not contain significant motion, the amount of the integration error is minimized.

The "no movement" in question is no movement between the treatment flashes (i.e., as part of the step) rather than no movement during the flash, which has already been assumed.

The speed signal is then integrated in step 102 to obtain a step size estimate. The next step 104 is to calculate the step size with respect to the treatment window location using a coordinate transformation. Based on this step estimate the treatment window speed is calculated in step 106.

The treatment window speed in turn is integrated again in step 108 to obtain a corrected treatment window step size estimate. An additional correction is needed to further improve estimation accuracy.

The final step 110 is to calculate the treatment window speed in the device coordinate system. This signal is needed to enable treatment direction detection.

Returning to Fig. 3A, the device movement direction with respect to the treatment window is determined in step 58. During this step, the device movement direction with respect to the treatment window is calculated by analyzing the x- and z- speed and step size signals. The x- and z-sensor axes are the 2 axes in line with the treatment window. These step size signals are used to determine the movement direction. One of the possibilities is to select the axis with the highest measured absolute speed as the movement direction. Another option is to choose the axis with the highest absolute step size as the movement direction. The direction of the movement along the dominant axis is also calculated (E. g. X-/X+/Z-/Z+).

In particular, it is determined if the device is moved in the horizontal (lateral) or vertical (longitudinal) direction of the device. This is for example needed because the treatment window may have different dimensions along the two axes (e.g., 1cm x 3cm) so knowing the movement direction enables overlap or gaps to be determined based on the detected step size.

In order to obtain an accurate step size estimate, it is important to select the correct amount of sensor data samples which need to be integrated. So, in addition to the "no movement" filter, an "end-of-step" and optionally a "beginning-of-step" index is calculated in step 60.

This is done in several steps:

First, the dominant movement direction axis speed signal is selected as the input for this calculation. The dominant direction detector output (e.g. horizontal or vertical movement) is used to determine which sensor axis contains the device movement signal. This signal (e.g. from the x- or z sensor axis) is input to the "end-of-step" detection algorithm.

The index of the maximum speed is determined within the signal. For example, if a step signal contains 100 samples., one of these samples contains the highest measured speed within the step. The index of this sample within the step signal is calculated (and will therefore be a number between 0 and 99).

The index of the maximum speed is then used as the starting point from which to look for the index/time point where the speed signal crosses the zero-speed point. This index/time point is defined as the step end index.: If such a zero-crossing does not exist, the index of the last sample is selected as step end index. This step end index is a point in time.

By default, the "beginning-of-step" index can be set to the first sample. This approach basically assumes that the user moves the IPL device directly after flashing so that movement occurs in the beginning of the step segment (and hence the integration can start as soon as the flash is over). Most users do this. However, there are users who start moving the device during the second part of the step signal. For this use case, a " beginning-of-step" can also be calculated. The "beginning-of-step" index can be set to the zero-crossing index of the speed signal prior to the "step end index", and to the first sample index if such a 0-crossing does not exist.

Moving to Fig. 3B, in step 62 a device rotation signal is calculated. For certain user scenarios (e.g., horizontal/vertical rotation, steps with rotation), the maximum device rotation angles (x/y and z sensor axes) within one step may be assessed. The rotation signal is calculated by integrating the gyro rotational speed signals (x, y and z axes).

In step 64. the signal quality is calculated. In order to make the algorithm more robust, a signal quality filter is applied to filter out steps with bad data. Bad data is usually caused by signal integration correction failures, insufficient movement or excessive movement. Signal quality is calculated by comparing the dominant movement axis step estimation signal with a good and a bad signal template. A score between 0 and 100% is calculated based on this comparison. By way of example, scores larger than 50% are considered good.

In step 66, the 1D step size estimate is obtained. The 1D step size is estimated as follows:

(i) Integrate the corrected 3D speed signal to obtain the 3D step size for this segment (i.e. for one step).
(ii) Find the dominant movement direction
(iii) Find the end of the displacement in the segment. This uses the step end index.
(iv) Calculate the 1D step size.

In step 68 a stroke detection algorithm is applied. The stroke detection algorithm examines the movement data to determine if the user started a new treatment stroke. There are two types of strokes: (i) regular stroke transitions where the user jumps from one end of the treated body part to the other end to start a new stroke in the same direction, and (ii) those where the user moves back and forth across the body part (which is referred to as a zigzag pattern).

For the regular stroke transitions, the maximum movement speed may be used for detection since the movement will be much faster than during treatment. Also, the measured displacement distance is taken into account. For example, this distance needs to be larger than a predefined threshold. When the maximum of the magnitude of the speed vector in x and z directions is more than a threshold t, a stroke transition is detected.

For a zigzag stroke transition, it is monitored if at least a minimum number (N) of steps in the opposite direction but along the same axis occurred. If that is the case, then it can also be concluded conclude that a stroke transition has happened N steps ago.

The algorithmic steps described above may be considered to be purely mathematical. A possible refinement is to refine the 1D step size estimate by applying a machine learning model as discussed above, in step 70.

The step size estimate as calculated using the mathematical algorithm may contains a large measurement error for individual steps. This error averages out to a much smaller error when taking multiple steps into account. However, it is also useful to have a good step-by-step size estimate. It has been found that an AI model can be trained to be capable of refining the step size estimate. The machine learning model may be a UNET model, comprising a combination of CNN and LSTM networks. The features used to train the network are:

Skin detection: 1 when there is contact with skin, 0 when not;
3D rotation in degrees as calculated based on the IMU sensor data;
3D speed signal in IMU sensor coordinates as calculated based on the IMU sensor data;
3D step size estimates in IMU sensor coordinates as calculated based on the IMU sensor data;
1D step estimate as calculated based on the IMU sensor data.

As with the regular data processing approach, the input data is partitioned into segments using the lamp flashes as boundaries. The resulting movement section data segments are offered to the network for training and prediction.

It has been found that averaging multiple step estimates results in a much more accurate step size estimate. Of course, this is at the expense of the real-time response of the system. Averaging may thus be performed as part of step 70.

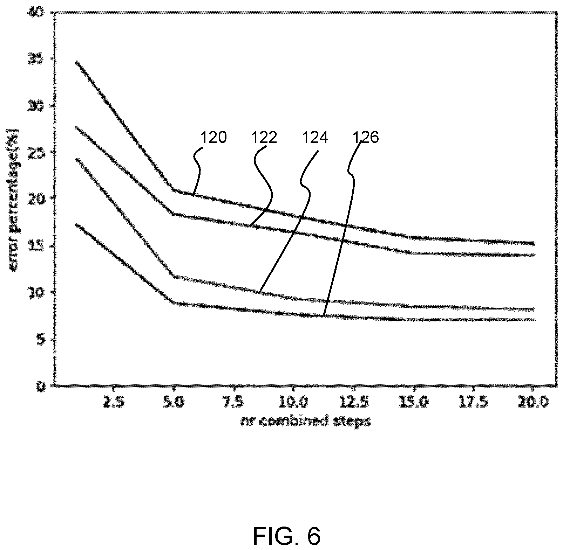

Fig. 6 shows the effect of averaging. It may be applied to both AI-refined and non-AI-refined estimates.

Fig. 6 plots an error percentage versus the number of steps being averaged. Plot 120 shows the mean step error without AI refinement, plot 122 shows the median step error without AI refinement, plot 124 shows the mean step error with AI refinement and plot 126 shows the median step error with AI refinement.

Choosing to average 5 estimates results in big jump in estimation performance. It is a good balance between real-time performance and estimation accuracy.

Returning to Fig. 3B, the next step is to perform usage pattern detection in step 72. Several usage patterns are defined that make it difficult for the user to achieve good coverage results or get good coaching / step size detection accuracy. Each usage pattern defined is detected using a tailored algorithm. Examples of different usage patterns and their detection algorithms are explained below:

(i) Horizontal - vertical rotation

The user may rotate the device by about 90 degrees from horizontal (lateral) to vertical (longitudinal). The coverage achieved is not properly defined for this step, so we do not want to coach based on this usage. This pattern is detected by checking the cumulative rotation of the sensor using the gyroscope data. If the total rotation during the step is larger than a minimum rotation threshold r1 (typically set to 45 degrees) and smaller than a maximum rotation threshold r2 (typically set to 135 degrees), the pattern is detected.

(ii) Upside down rotation

The user may rotate the device by about 180 degrees, It is more difficult for the user to make precise device placement after such a rotation. The large rotation with accompanying large accelerations and rotation speeds, makes it very hard to accurately detect the step size. This pattern is detected by checking the cumulative rotation of the sensor using the gyroscope data. If the total rotation during the step is larger than a minimum rotation threshold r2 (typically set to 135 degrees) and smaller than a maximum rotation threshold r3 the pattern is detected.

(iii) Movement of body part

The user may move the body part that is being treated. Movement of the body cannot be measured by the device sensor, so any step size estimate will be inaccurate. It has been observed that during regular treatment without body movement the device locations when placed on the skin are located in a stable plane parallel to the skin. When the device makes a step away from that plane, it can only stay on the skin when the body part moves as well. To detect these types of body movements, the maximum displacement in the y-direction of the sensor (se Fig. 1) can be estimated during the step, as well as the percentage of time the sensor is on the skin. If the maximum displacement is larger than a threshold t (e.g., 5cm) and more than p% (e.g., 80%) of the time the sensor is on the skin, then the body part has moved. As a movement larger than t2 (e.g., 50cm) is not to be expected because of physical constraints, we only detect a body movement if the displacement is smaller than t2.

(iv) Lifting off skin

The user may lift the device off the skin. It is more difficult for the user to make precise movements, and more difficult for the sensor to estimate the step size across the skin. A lift off the skin is detected when the maximum displacement in the y-direction of the sensor is at least d1 (e.g., 1.5cm) and the percentage of skin contact time is less than s% (e.g., 80%).

(v) Stroke transition

The user will perform multiple strokes. When the user moves to a new stroke, the step size of this transition should not to be coached upon.

(vi) Movement too slow

The user may move the device too slowly for the sensor to measure anything useful. When the user makes a clearly defined step movement, the 1D displacement signal has a slope from one displacement value to another and then remains stable at that new displacement value. If the movement is too slow to measure accurately, the signal is a more straight line. Both shapes can be fitted to the 1D displacement signal, and the fit quality of both can be used to decide if the movement was sufficiently clear. If the straight line fits better than a step sloped step shape, the movement was not sufficiently clear.

(vii) Steps with rotation

The user may rotate the device too much during the step. This makes it more difficult for the sensor to accurately measure step size. This can be detected by measuring the maximum rotation across all axes at any moment during the step. If this maximum rotation is larger than a threshold m1 (e.g., 10 degrees) and smaller than the threshold r1, this usage pattern can be detected. (viii) Searching behavior

The user may take very long to trigger the next flash. This makes it more difficult to estimate step size. An expected step duration can be estimated based on previous steps, and if the current step size is larger than s2 (e.g. 2) times the expected step duration, it can be concluded that there is searching behavior.

Segments with unreliable step size estimates, for the reasons explained above, can be discarded and hence not form part of the assessment of the performance. These form the outlier-use cases referred to above.

After computing the step size and discarding unreliable segments, a coverage percentage can be obtained in step 74 based on the estimated direction of the movement of the device. This coverage percentage is part of a treatment report presented in step 76.

As mentioned above, the step size data and other detected behavior and coaching must be communicated to the user in a way that is easily understandable. This feedback can be given during the treatment and after the treatment. Users are typically not interested to know the exact step size in millimeters, they just want to know if they have missed any spots or if they have over-coverage (i.e., multiple flashes on the same area). One way to achieve this is to translate the distance in mm of the step into this coverage percentage (for that step). This is the travelled distance divided by the effective size of the treatment window in the movement direction:

$$\text{Coverage \%} = (100 * \text{effective treatment window size}) / \text{traveled distance}$$

As the device typically has a rectangular flashing surface of about 1cm by about 3cm, the user has to move the device by about 1cm in the one direction to get perfect coverage, and by about 3cm in the other direction to get perfect coverage. So, for good coaching, it is necessary to determine the direction of movement (lateral or longitudinal). The first guess of the movement direction is based on the estimated displacement in the x and z direction. If the displacement in the x direction is largest it can be concluded that movement is in one direction and in the other direction otherwise. After the first guess, the final direction may be obtained by using a majority vote over all steps since the last horizontal/vertical rotation or stroke transition.

To enable near real-time feedback on the step size, it is important to have fast computations. To enable this all computations are done on a per-step basis. Therefore, sensor data is collected until a full step is available, and then this step is processed. After the step has been processed, the original sensor data is no longer needed and is removed. The result of the processing is the step size estimate, the direction of movement, and which usage patterns were found in the step. Only this data is saved for later use and is sufficient to recompute all results needed for coaching. As a result, the processing time is greatly reduced compared to repeated processing the full set of sensor data.

The treatment report may include various information such as:

(i) The length of each stroke/set of strokes
(ii) The number of strokes per treatment
(iii) The average stroke length per treatment
(iv) The step size estimate of each individual step or for a set of x steps.
(v) The average step size for each stroke or for a set of strokes.
(vi) The number of steps per stroke or for a set of strokes

When performing a treatment, users have to pay attention to many things, and an even simpler way of communicating coverage is preferred to a numerical percentage. This can for example be an output in the form of colors, for example: blue means missed spots, yellow/orange is double flashing, and green is good. The percentages for determining the color can be any suitable level, such as <80%, 80-120% and >120%. This means that the coverage doesn't have to be exactly 100% for it to be counted as "good", otherwise it would be very difficult to reach this which can be very frustrating for users and could cause them to stop using the coaching feature.

During a treatment, it is not realistic to expect users to watch an app screen all the time. The user interface may for example be simplified by using large color elements in the real-time feedback screens, which can already be seen from the corner of the eye. Voice feedback can also be added that can be heard even when not looking at the screen.

For example, a screen may have a large blue region, and the audio may advise "missed spots". The screen may have a large green region, and the audio may advise "good ". Finally, the screen may have a yellow region, and the audio may advise "overlap". The user may be able to interact with the screen to request further details.

Of course, having a new screen output and a new voice message after every step is not desirable. During testing it has been found that users prefer the screen and voice messages to be updated approximately every 5 flashes. The feedback is then based on the average coverage of those last 5 steps.

It is also possible to give such feedback on the hand-held IPL device itself, e.g. with a small display or colored LED(s), and even with a speaker in the device.

Apart from real-time feedback, the system can show the user summaries of their coverage/performance after the treatment. These treatments can for example be stored for later reference, and provide useful insights that cannot be achieved during real-time feedback. A treatment is made up of several lines (strokes), sometimes more than 10 for example for a leg. It is useful to not only show a summary of the whole treatment, but also of the individual lines.

An easy way to provide a summary would be to show the average coverage of the whole line or the whole treatment. However, such an average coverage is not always telling the full story. For instance, a person can have over-coverage during the first part of a treatment, and under-coverage during the second part, which could result in an average coverage of 100% (green). It is for example better to show how many steps had under-coverage, how many had good coverage, and how many had over-coverage. This can be done with a table, histogram, or pie chart.

Instead of creating such a histogram based on all individual steps, it can also be based on the timing of the real-time feedback messages. This avoids that a certain total under-coverage percentage is reported when this was never mentioned during real-time feedback (e.g. because it was never significant enough to have an impact on the 5-step average). See below example screens for a line report and a treatment report.

The output interface is controlled to provide information to the user in an easily digestible manner. The feedback is for example provided on a screen of the smartphone.

A first type of feedback is a report for one line (stroke) of leg treatment. A representation of the leg may be provided with color coding at different heights along the leg for "good", "missed parts" or "overlap". An overall summary may also be provided, such as a rating (e.g., "Good") and an explanation (e.g., "Your technique is good. Keep it up in your next line,")..

A second type of feedback is a report for a full treatment session. Again, an overall summary is provided with a rating (e.g, Well gone!) and an explanation (e.g, "You completed the first leg treatment on time and with good coverage"). An overall report on progress of a treatment program may be made, with an indication of when the next treatments are due. For the treatment that has been completed, a total number of flashes may be reported together with a breakdown of the individual flash coverage into "good", "missed spots" and "overlap". These may for example be shown as percentages on a pie chart. An overall duration may also be provided.

As described above, the algorithm cannot cope well with some types of movements like stamp-and-flash, leg movements, etc., referred to above as edge cases. If the rejection of steps occurs very often, there will not be enough valid step size estimations for proper user feedback about coverage. In those cases, the user should receive feedback on those unsupported usage patterns. For instance, when too many stamp-and-flash movements were made in a row, the app (or other UI execution) may show a message about this to the user (accompanied with audio feedback).

Because users can only process so much information at a time, the coverage feedback and usage pattern feedback may be separate, for example showing only one or the other at each (5 step) coaching interval. Important usage pattern feedback should always get priority. In other words: users should first be trained on usage technique, and then on coverage.

During real time guidance, messages may be provided for example:

asking the user to keep their leg still; or
asking the user to keep skin contact; or
asking the user to move the device more consistently.

This feedback may be visual and/or audible, and the user may of course choose to turn the audio function on and off.

The system may be enhanced with additional sensors. For example, a second motion sensor may be provided for detecting motion relative to the user's skin. In combination with the motion sensing (and the assumed zero relative motion during the flash) this can provide increased robustness to body movement. An optical IR sensor or camera may be used for this purpose.

The use of the device described above involves the user actuating a trigger to provide a light flash. Another option is to flash automatically when the moved distance is the correct amount. This may be implemented making use of an accurate skin location detection system, such as a camera.

Another option is to control release of a flash, when instructed by the user by means of the trigger, when the step size is appropriate. In this case, the user moves by a step, presses the trigger, and the system then computes if the step is the correct size and only then releases the flash. If not, a correction may be suggested, and the new step may then be measured and added to the previous step to decide if the flash should be released at the new location.

Thus, in addition to the coaching function, there is also the option to directly influence (or allow) the flash.

As explained above, steps may be discarded when they are for special use cases. These step size for these steps may be replaced by an average of a previous number of steps.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

# EP 4 537 778 A1

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An IPL system for delivering IPL light to a user's skin during IPL treatment, comprising:

   an IPL device (10) comprising:

   a light source (16);
   a light delivery window (18);
   a controller (20) for controlling the light source to deliver light flashes to the light delivery window and for reporting a timing of the light flashes as light flash timing; and
   a motion sensor (22) for sensing motion of the IPL device;

   a processor (20) for processing the motion sensor output and the light flash timing; and
   an output interface (24; 30),
   wherein the processor (20) is configured to:

   derive a step size of movement of the device between light flashes from the motion sensor output and the light flash timing; and
   control the output interface to provide feedback information to the user relating to the step size.

2. The system of claim 1, wherein the processor (20) is configured to assume a zero movement of the IPL device during the light flash.

3. The system of claim 1 or 2, wherein the processor (20) is configured to control the output interface to provide user coaching in respect of the correct amount of movement between flashes to achieve skin coverage.

4. The system of any one of claims 1 to 3, wherein the IPL device comprises a skin detection sensor (26), wherein the processor (20) is further configured to detect when the device is placed on the skin.

5. The system of claim 4, wherein the processor (20) is configured to control the output interface to provide user coaching in respect of one or more of:

   correct application of the light delivery window to the skin during movement between flashes;
   correct orientation of the light delivery window to the skin during movement between flashes;
   sufficiently small movement of the user during the IPL treatment.

6. The system of any one of claims 1 to 5, wherein the processor (20) is configured to:

   derive a 3D linear acceleration by removing a gravity component from the motion sensor output;
   integrate the 3D linear acceleration to derive a 3D velocity assuming a zero movement during the light flashes;
   integrate the 3D velocity to derive a step size estimate between flashes.

7. The system of claim 6, wherein the processor is further configured to provide the step size estimates to a machine learning algorithm thereby to obtain a refined step size estimate.

8. The system of any one of claims 1 to 7, wherein the motion sensor (22) comprises a 6-axis inertial monitoring unit, IMU.

9. The system of any one of claims 1 to 8, further comprising a second motion sensor for detecting motion relative to the user's skin.

10. A method of processing a motion sensing signal and a light flash timing signal obtained during IPL treatment using an IPL device, the method comprising:

deriving a step size of movement of the device between light flashes from the motion sensor output and the light flash timing signal; and
controlling an output interface to provide feedback information to the user relating to the step size.

11. The method of claim 10, comprising:
assuming a zero movement of the IPL device during the light flash.

12. The method of claim 10 or 11, comprising:

controlling the output interface to provide user coaching in respect of the correct amount of movement between flashes to achieve skin coverage; and/or
receiving a skin contact detection signal obtained during IPL treatment and controlling the output interface to provide user coaching in respect correct application of the light delivery window to the skin during movement between flashes.

13. The method of any one of claims 10 to 12, comprising:

deriving a 3D linear acceleration by removing a gravity component from the motion sensor output;
integrating the 3D linear acceleration to derive a 3D velocity assuming a zero movement during the light flashes;
integrating the 3D velocity to derive a step size estimate between flashes.

14. The method of claim 13, further comprising providing the step size estimates to a machine learning algorithm thereby to obtain a refined step size estimate.

15. A computer program comprising computer program code which is adapted, when said program is run on a computer, to implement the method of any one of claims 10 to 14.

FIG. 1

FIG. 2

capture step sensor data section — 50

Execute sensor fusion algorithm — 52

Calculate device speed — 54

Calculate 3d step size estimate — 56

Calculate device movement direction w.r.t. treatment window — 58

Calculate step end index — 60

FIG. 3A

```
              ┌─────────────────────────────────────┐
              │  Calculate device rotation signal   │───── 62
              └─────────────────────────────────────┘
                            │
                            ▼
              ┌─────────────────────────────────────┐
              │       Calculate signal quality      │───── 64
              └─────────────────────────────────────┘
                            │
                            ▼
              ┌─────────────────────────────────────┐
              │      Calculate 1d step size estimate│───── 66
              └─────────────────────────────────────┘
                            │
                            ▼
              ┌─────────────────────────────────────┐
              │    Execute stroke/new line detection│───── 68
              └─────────────────────────────────────┘
                            │
                            ▼
              ┌─────────────────────────────────────┐
              │     Execute AI step size refinement │───── 70
              └─────────────────────────────────────┘
                            │
                            ▼
              ┌─────────────────────────────────────┐
              │ Execute use case detection (e.g.    │───── 72
              │ stamp/flash, horizontal/vertical    │
              │ rotation, movement leg)             │
              └─────────────────────────────────────┘
                            │
                            ▼
              ┌─────────────────────────────────────┐
              │          Calculate coverage         │───── 74
              └─────────────────────────────────────┘
                            │
                            ▼
              ┌─────────────────────────────────────┐
              │       Generate treatment report     │───── 76
              └─────────────────────────────────────┘
                            │
                            ▼
                            ⊗
```

FIG. 3B

FIG. 4

FIG. 5

FIG. 6

Europäisches
Patentamt

European
Patent Office

Office européen
des brevets

# EUROPEAN SEARCH REPORT

**Application Number**

EP 23 20 3485

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/172663 A1 (KOOIJMAN GERBEN [NL] ET AL) 8 June 2023 (2023-06-08) * paragraphs [0011], [0014], [0023], [0049], [0052], [0058], [0067], [0070], [0075] – [0079], [0086], [0092], [0106]; figures 1a,1b,3,5 * | 1-15 | INV. A61B18/18 |
| X | US 2021/322098 A1 (BOURQUIN YANNYK PARULIAN JULIAN [NL] ET AL) 21 October 2021 (2021-10-21) * paragraphs [0028], [0041], [0045], [0065], [0072], [0073], [0095], [0107], [0108], [0112]; figures 1-4 * | 1-3,5-15 | |
| X | EP 4 193 950 A1 (KONINKLIJKE PHILIPS NV [NL]) 14 June 2023 (2023-06-14) * paragraphs [0019] – [0028], [0043], [0103], [1005]; figures 1,3,4 * | 1-15 | |
| X | US 2023/015956 A1 (FERNANDO SHAKITH DEVINDA [NL] ET AL) 19 January 2023 (2023-01-19) * paragraphs [0048], [0052], [0053], [0064] – [0067]; figures 1,3 * | 1-15 | **TECHNICAL FIELDS SEARCHED (IPC)** A61B |

The present search report has been drawn up for all claims

| Place of search | Date of completion of the search | Examiner |
|---|---|---|
| Munich | 26 February 2024 | Link, Tatiana |

EPO FORM 1503 03.82 (P04C01)

## ANNEX TO THE EUROPEAN SEARCH REPORT
## ON EUROPEAN PATENT APPLICATION NO.

EP 23 20 3485

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2024

| Patent document cited in search report | | | Publication date | Patent family member(s) | | | Publication date |
|---|---|---|---|---|---|---|---|
| US 2023172663 | A1 | | 08-06-2023 | CN | 115666433 | A | 31-01-2023 |
| | | | | EP | 3915502 | A1 | 01-12-2021 |
| | | | | EP | 4157125 | A1 | 05-04-2023 |
| | | | | JP | 2023526992 | A | 26-06-2023 |
| | | | | KR | 20230016667 | A | 02-02-2023 |
| | | | | US | 2023172663 | A1 | 08-06-2023 |
| | | | | WO | 2021239529 | A1 | 02-12-2021 |
| US 2021322098 | A1 | | 21-10-2021 | CN | 112584788 | A | 30-03-2021 |
| | | | | EP | 3613376 | A1 | 26-02-2020 |
| | | | | EP | 3840679 | A1 | 30-06-2021 |
| | | | | ES | 2914395 | T3 | 10-06-2022 |
| | | | | JP | 7420789 | B2 | 23-01-2024 |
| | | | | JP | 2022501091 | A | 06-01-2022 |
| | | | | KR | 20210050532 | A | 07-05-2021 |
| | | | | PL | 3840679 | T3 | 11-07-2022 |
| | | | | US | 2021322098 | A1 | 21-10-2021 |
| | | | | WO | 2020038970 | A1 | 27-02-2020 |
| EP 4193950 | A1 | | 14-06-2023 | EP | 4193950 | A1 | 14-06-2023 |
| | | | | WO | 2023104587 | A1 | 15-06-2023 |
| US 2023015956 | A1 | | 19-01-2023 | BR | 112022012319 | A2 | 06-09-2022 |
| | | | | CN | 114845653 | A | 02-08-2022 |
| | | | | EP | 3842002 | A1 | 30-06-2021 |
| | | | | EP | 4081149 | A1 | 02-11-2022 |
| | | | | IL | 294165 | A | 01-08-2022 |
| | | | | JP | 2023510153 | A | 13-03-2023 |
| | | | | KR | 20220121248 | A | 31-08-2022 |
| | | | | US | 2023015956 | A1 | 19-01-2023 |
| | | | | WO | 2021130052 | A1 | 01-07-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3621775 A
- EP 3938155 A
- WO 2017002004 A
- WO 2016174621 A

**Non-patent literature cited in the description**

- **SEBASTIAN MADGWICK**. *An efficient orientation filter for inertial and inertial/magnetic sensor arrays*, 30 April 2010, http://www.x-io.co.uk/open-source-imu-and-ahrs-algorithms